# EUROPEAN PATENT SPECIFICATION

(11) **EP 4 630 068 B1**
(45) Date of publication and mention of the grant of the patent: **02.09.2026**
(21) Application number: 23821533.9
(22) Date of filing: 05.12.2023
(51) Int. Cl.: A61L 27/44, A61L 27/46

(54) **A PROCESS FOR MANUFACTURING A RESORBABLE COMPOSITE BIOMATERIAL, THE RESORBABLE BIOMATERIAL PRODUCED, AND ITS USE IN VARIOUS APPLICATIONS**
VERFAHREN ZUR HERSTELLUNG EINES RESORBIERBAREN ZUSAMMENGESETZTEN BIOMATERIALS, DAS HERGESTELLTE RESORBIERBARE BIOMATERIAL UND SEINE VERWENDUNG IN VERSCHIEDENEN ANWENDUNGEN
PROCÉDÉ DE FABRICATION D'UN BIOMATÉRIAU COMPOSITE RÉSORBABLE, BIOMATÉRIAU RÉSORBABLE PRODUIT ET SON UTILISATION DANS DIVERSES APPLICATIONS

(30) Priority: 06.12.2022 EP 22306793
(43) Date of publication of application: 15.10.2025
(73) Proprietor: UNIVERSITE CLAUDE BERNARD - LYON 1, 69100 Villeurbanne (FR); Centre National de la Recherche Scientifique, 75016 Paris (FR); Institut National des Sciences Appliquées de Lyon (Insa Lyon), 69100 Villeurbanne (FR)
(72) Inventor: LACAMBRA, Xavier, 25005 Lleida Lleida (ES); CHENAL, Jean-Marc, 69100 VILLEURBANNE (FR); LAME, Olivier, 69001 LYON (FR)
(74) Representative: IPAZ
(86) International application number: PCT/EP2023/084210
(87) International publication number: WO 2024/121092

(56) References cited:
- US-A1- 2022 080 630

## Description

The present invention relates to a process for manufacturing a composite biomaterial comprising at least one biocompatible resorbable organic polymer and at least one bioactive inorganic material, a composite biomaterial obtained by said process, an implantable device comprising said composite biomaterial, said composite biomaterial for medical use, or for use as a bone substitute or to support bone regeneration, and a sterilization process implementing said composite biomaterial.

Bone is a unique tissue that is capable of repairing itself after damage. However, there are certain instances of fractures, and defects which can result from trauma, cancers, infections and degenerative and inflammatory conditions, that require a medical intervention to install an internal fixation. This system provides a temporary support to help the bone to restore the full function, ensure a correct alignment of fractured bones and minimize the possible complications during the healing. As with any implant, careful consideration of the material used to create the implants to treat these problems is needed. If the incorrect material is chosen, the implants themselves can lead to bone fractures or defects, or bone healing may not take place at all. Consequently, the design of implants requires consideration of the material's biocompatibility, mechanical properties, and surface properties as well as its chemical properties and failure properties so that the implant closely parallels the biomechanical properties of bone and integrates with the native tissue while maintaining its integrity for the requisite duration.

The production and quality of biomaterials have grown exponentially in the last years with strong clinical outcomes in all medical disciplines, a market which potentially has a global size: in fact, damages to tissues following traumas, neoplasms, congenital defects joined to wider problems, such as post-extraction bone gaps in dentistry and bacterial infections to bone tissues account for big expenses for National Health Systems. Moreover, this is a continuously growing market, because the social and economic impacts of pathologies linked to the muscle-skeleton apparatus become more and more important if degenerative pathologies are considered, more and more frequent following the increase of life expectancy and of the progressive ageing of populations in developed Countries.

In traumatology and orthopedic surgery, the use of "inert" biomaterials like metallic osteosynthesis systems have shown good clinical results for the fixation of bone fragments since they present biocompatibility and a mechanical stability during the fracture healing. However, the difference between the implant and the bone stiffness may produce a stress-shielding effect leading to bone degradation and aseptic loosening. Metallic implants are known to cause artefacts on computed tomography imaging, perturbing the clear observation of the implant and the surrounding bone. Moreover, long-term implantation of "inert" biomaterials fixation presented several disadvantages such as inflammatory responses in the surrounding tissues or allergic reactions induced by the product of metallic corrosion. After the completely fracture healing, a second surgical operation is normally required to remove the non-resorbable implants, such surgical procedure can induce possible clinical complications, such as for example the risk of bacterial infections for patients. The aforementioned problems may be prevented by using biocompatible resorbable polymer-based devices.

Hence, the new generation of orthopedic implants should be biocompatible, biodegradable, mechanically stable and promote cell attachment and proliferation. Such implants can be ceramics, polymers synthesized or natural, or a combination of these materials in the form of composites.

Recently, the scientific community has shown an increasing interest on biodegradable synthetic polymer-ceramic composites and the corresponding manufacturing process for bone tissue engineering. Among the latest generation of biomedical grade composites, some combine inorganic fillers and a polyester matrix in order to obtain osteoinductive, osteoconductive and resorbable materials. Calcium phosphates and bioactive glasses (the main bioactive fillers used in orthopedic applications) present the advantage of buffering the acidic degradation of the polyester matrix, and thus prevent from or at least reduce an inflammatory response from host tissues. However, a frequently and particularly critical problem (for the control of final properties) during the manufacturing of bioglass-based composites, is the hydrolytic degradation of the polyester matrix catalyzed by the filler and the thermomechanical constraints associated to the process. For example, *in-vitro* investigations into the degradation of such composites have shown a significant loss of weight, molar mass, and mechanical strength from the first week of immersion in a phosphate buffer saline solution (PBS). Filler particles (especially bioactive resorbable fillers such as bioglass) indeed accelerates the polymer matrix degradation when immersed in such PBS solution. As a result, for some applications, the degradation can take place faster than expected and the medical device can loss the mechanical properties and mass before the complete bone healing. Additionally, in some cases, the hydrolytic degradation can cause an inflammatory response of surrounding tissues. Besides, at least for medical applications, these biomaterials are systematically submitted to a sterilization step. The gamma-irradiation sterilization technique is the most widely used technique for orthopedic implants sterilization. However, such technique increases the polymer matrix degradation.

In the literature, biodegradable synthetic polymer-ceramic composites have been processed mainly through two different techniques: dissolution and extrusion. In the dissolution technique, the polymer matrix and filler particles are blended thanks to a solvent. Afterwards, the resulting solution is precipitated and the composite is dried and ground into pellets. These pellets can be transformed into implants using a conventional thermomechanical process such as injection molding, injection transfer molding, compression molding, extrusion or microtechnical machining. The international application WO2008/116984 describes such type of technique. This entire process is time consuming and rather inconvenient from an industrial viewpoint. It involves solvent(s) which should be avoid as much as possible for toxicity issues (toxic chemical substances may still be present in the final product) and scale-up (some polymer solvents present significant obstacles to make the transition from laboratory to industrial application).

The second technique involves melt blending at elevated temperatures using extrusion, followed by injection molding of the implants. Several reports describe bubble formation and the coloration of composites during the processing of composites by this second technique. In addition, at high temperatures, a chemical reaction occurs between the silicate functions on the surface of the filler and ester groups of the polyester accelerating the hydrolytic degradation. Through the last years, several studies have tried to reduce this chemical reaction by coating the filler surface with a resorbable polymer, or varying the filler composition, size and shape, or very recently applying a thermal treatment on filler particles. As an example, Lacambra-Andreu et al. [Polymers, 2021, 13, 2991, 1-17] describe the preparation of PDLA (polylactide) / bioglass composite by heat-treating the filler at a temperature ranging from 580 to 800°C, dry-mixing the heat-treated filler with PDLA, and extruding and injection-molding the resulting mixture under argon. Thermally treating the filler and/or optimizing its size helps controlling the degradation of the polymer during the composite manufacting process. Nevertheless, the mechanical properties of the final product are not optimized and degragation remains important.

US2022080630 discloses a method for forming a thermoplastic composite body having regions with varied material composition and/or porosity . The parts are assembled and compression molded at elevated temperature sufficient to melt and fuse polymer particles or parts with minimal damage to the reinforcement particles.

Thus, the aim of the present invention is to overcome the drawbacks of the cited prior art, and more particularly, to provide a composite biomaterial which displays a limited or reduced hydrolytic degradation of the polymer matrix, and/or which has improved mechanical properties, so that it can be used as a bone substitute and/or as part of an implant device. Another aim of the present invention is to provide a process which leads to the above-described composite biomaterial, namely a resorbable composite biomaterial displaying a limited or reduced hydrolytic degradation of the polymer matrix during manufacturing process, and/or having improved mechanical properties, said process being simple, easy to scale up and limiting or at least reducing the use of solvents.

### The process

A first object of the present invention is a process for manufacturing a composite biomaterial comprising at least one biocompatible resorbable organic polymer and at least one bioactive inorganic material,
wherein said process comprises at least the following steps:
i) compacting a powdery solid composition comprising at least one biocompatible resorbable organic polymer and at least one bioactive inorganic material, with a compaction pressure of at least about 2 MPa, so as to form a compacted solid composition, said biocompatible resorbable organic polymer being defined by a glass transition temperature Tg, and having a cristallinity degree of at most 30%,
ii) sintering the compacted solid composition of step i) at a temperature Ts of at most Tg + 70°C, and at a holding pressure greater than atmospheric pressure, so as to form a sintered solid, and
iii) cooling the sintered solid.

The inventors have surprisingly found that thanks to the sintering process of the present invention, a composite biomaterial having improved mechanical properties and/or improved stability can be obtained. As a result, the composite biomaterial can be used in various medical applications which requires such properties as for example bone healing, but also in various other applications where composite biomaterials comprising an inorganic filler and a polymer matrix are expected and the stability of said polymer matrix and/or the mechanical properties of said composite biomaterials are an issue.

In particular, contrary to processes of the prior art, the process of the present invention does not cause a significant reduction of the molar mass of the polymer matrix and of the mechanical properties of the final composite biomaterial. In particular, the sintering step ii) is carried out at lower temperatures compared to well-known plastics processes (molding-injection, extrusion, etc...) so that the molar mass of the polymer matrix and the mechanical properties of the final composite biomaterial are preserved as much as possible.

Additionally, contrary to other processes for manufacturing composite biomaterials described in the prior art, the process of the present invention leads to a composite biomaterial which can be sterilized with significantly less aggressive methods than gamma-irradiation, such as ethylene oxide or X rays sterilization, because the manufacturing process of the invention garantees the absence of internal stress in the composite biomaterial
The process of the present invention comprises two distinct steps i) and ii):
- the first, rapid step i) eliminates the "voids" between the grains, by deforming them by crushing involved by compaction, in order to maximise the interface between the grains, and
- the second step ii), which takes more or less time, allows the polymer chains to interdiffuse at the interfaces between neighbouring grains and to obtain a solid sample without passing through the molten (liquid) state of the polymer.

### Step i)

The first step i) corresponds to a densification step i). It enables the coalescence of particles in step ii).

### The biocompatible resorbable organic polymer

In the present invention, the expression "polymer" means a homopolymer or a copolymer.

The biocompatible organic polymer is resorbable.

In the present invention, the term "resorbable" means that the biocompatible resorbable organic polymer has ability to degrade and be absorbed by the human or animal body. In other words, the biocompatible organic polymer can be broken down or digested by microorganisms (e.g. bacteria, fungi, algae), especially by the action of enzymes. The reactions involved during biodegradation in humans or animals are hydrolysis reactions, that is to say the breaking of covalent bonds by reaction with water (cf. current standard NF EN 13432).

More particularly, the resorbable biocompatible organic polymer should preferably have a molar mass higher than 40 000 g/mol, after being immersed in phosphate-buffered saline solution (PBS solution) under stirring at 37°C up to 120 days.

The resorbable organic polymer is biocompatible.

In the present invention, the term "biocompatible" means that the biocompatible resorbable organic polymer is compatible with living tissue. In other words, the resorbable organic polymer does not interfere with, and does not degrade, the biological environment in which it is used; it does not produce any toxic, inflammatory, or immunological reaction when exposed to the body or body fluids.

The biocompatible resorbable polymer is organic.

In the present invention, the term "organic" means that the biocompatible resorbable organic polymer essentially contains carbon atoms, hydrogen atoms, and optionally heteroatoms selected from the group consisting of oxygen, nitrogen, sulfur atoms, and mixture thereof.

The biocompatible resorbable organic polymer is defined by a glass transition temperature Tg and a cristallinity degree of less than or equal to about 30% (i.e. at most about 30%). In other words, the biocompatible resorbable organic polymer can be an amorphous polymer or a semi-cristalline polymer. A cristallinity degree of less than or equal to about 30% may limit the release of cristals which induce inflammatory responses in the surrounding tissues during implantation.

Preferably, the biocompatible resorbable organic polymer has a glass transition temperature Tg ranging from about -60°C to about 100°C, more preferably about -20°C to about 70°C, and even more preferably from about 45°C to about 65°C.

In the present invention, the glass transition temperature Tg can be measured by techniques well known to those skilled in the art, such as Differential Scanning Calorimetry (DSC) measurements, in particular with a heating rate of 10°C/min under nitrogen.

The biocompatible resorbable organic polymer has a cristallinity degree of at most about 30%, preferably of at most about 20%, and more preferably of at most about 10%. Thanks to such low cristallinities, the compacted solid composition can be sintered at lower temperatures which limits hydrolytic degradation during the manufacturing process.

In one preferred embodiment, the biocompatible resorbable organic polymer is an amorphous polymer (i.e. cristallinity degree of less than 5%).

In the present invention the cristallinity degree is measured by methods well known to those skilled in the art, and more particularly by differential scanning calorimetry (DSC).

The biocompatible resorbable organic polymer can have a weight-average molar mass, Mw, ranging from about 40 000 g/mol to about 1 000 000 g/mol, preferably from about 90 000 g/mol to about 900 000 g/mol, and more preferably from about 100 000 g/mol to about 800 000 g/mol.

In the present invention, the weight-average molar mass is measured according to methods well known to those skilled in the art, and preferably by size exclusion chromatography (SEC) measurement (preferably in chloroform).

The biocompatible resorbable organic polymer can be selected from aliphatic polyesters, aliphatic polycarbonates, gelatins, and collagens.

Examples of aliphatic polyesters can include polyglycolides (polyglycolic acid or PGA), polylactides (polylactic acid or PLA), copolymers of lactic acid and glycolic acid (PLGA), polylactones (e.g poly(ε-caprolactone)), and polyhydroxyalcanoates (e.g. polyhydroxyvalerate, poly(hydroxybutyrate)).

Examples of aliphatic polycarbonates include poly(trimethylene carbonate) and its copolymer.

The biocompatible resorbable organic polymer is preferably an aliphatic polyester, and more preferably a poly(α-hydroxy ester), advantageously selected from polyglycolides (polyglycolic acid or PGA), polylactides (polylactic acid or PLA), and copolymers of lactic acid and glycolic acid (PLGA).

In the powdery solid composition (i.e. before compacting step i)), the biocompatible resorbable organic polymer is in the form of a powder, preferably having a particle size ranging from about 0.01 µm to about 3 mm, preferably from about 0.1 µm to about 500 µm, and more preferably from about 1 µm to about 200 µm. Above 3 mm, the composite biomaterial may have reduced homogeneity and bioactivity.

In the present invention, the particle size of the biocompatible resorbable organic polymer is measured according to methods well known to those skilled in the art, and preferably by Particle Size Analyser or Scanning Electron Microscopy.

The powdery solid composition can comprise one or more biocompatible resorbable organic polymers having the above-mentioned features.

When the powdery solid composition comprises several biocompatible resorbable organic polymers (blends) having respectively glass transition temperature Tg1, Tg2, Tg3, etc..., the temperature to be considered so as to define Ts may be the glass transition temperature which corresponds to the continuous polymer phase of the blend.

### The bioactive inorganic material

The inorganic material is bioactive.

In the present invention, the term "bioactive" means that the bioactive inorganic material has at least osteocondutive properties, and preferably osteoconductive and osteoinductive properties.

Osteoconduction represents the passive property of a material to receive bone regrowth, by vascular and cellular invasion. The link between said material and recipient tissue is then guaranteed by tissue growth (within the implant).

Osteoinduction represents the capacity of a material to induce cellular differentiation in order to synthesize a mineralized bone matrix.

Preferably, the composite biomaterial of the present invention is able to induce a specific biological response at the surface of the composite biomaterial to create a bioactive fixation at the composite biomaterial/host tissue interface.

The inorganic material can be resorbable or non-resorbable.

In the present invention, the term "resorbable" means that the inorganic material can disappear naturally by solubilization and/or be transformed into carbonated hydroxyapatite (a very similar formulation to bone).

The bioactive inorganic material can be selected from a bioactive glass (BAG), amorphous calcium phosphate (ACP), hydroxyapatite (HA), tri-calcium phosphate (TCP), tetra-calcium phosphate (TTCP), monocalcium phosphate (MCP), dicalcium phosphate (DCP), calcium silicate (CS), and Mg alloy.

In one preferred embodiment, the bioactive inorganic material has is in the form of spherical particles, fibers, or platelets.

In one preferred embodiment, the bioactive inorganic material has a specific surface area, preferably measured according to the well-known BET method, ranging from about 0.1 m²/g to about 200 m²/g. Such low specific surface areas may limit composite biomaterial degradation.

Examples of bioactive glasses include silicate-based bioactive glasses, borate-based bioactive glasses, and phosphate-based bioactive glasses.

In one preferred embodiment, the bioactive inorganic material is a bioactive glass. Bioactive glasses enable the formation of bone tissue. In particular, bioactive glasses are able to develop a link with the organic structures composing the connective tissue and the bone tissue, the collagen fibers, and to induce the mineralization of these latter ones with the activation of the progenitor cells of bones and the supply of a mineral substance, carbonated hydroxyapatite, similar as composition and structure to the mineral phase of a bone.

The bioactive glass can comprise, with respect to the total mass of said bioactive glass, at least:
- from about 40% by mass to about 60% by mass of SiO₂,
- from about 20% by mass to about 50% by mass of CaO,
- from about 0% by mass to about 30% by mass of Na₂O,
- from about 1% by mass to about 10% by mass of P₂O₅.

Silicate-based bioactive glasses are preferred. Silicate-based bioactive glasses display improved bioactivity.

In the powdery solid composition (i.e. before compacting step i)), the bioactive inorganic material is in the form of a powder, preferably having a particle size ranging from about 0.01 µm to about 3 mm, preferably from about 0.1 µm to about 500 µm, and more preferably from about 1 µm to about 100 µm. Above 3 mm, the composite biomaterial may have reduced homogeneity and bioactivity.

In the present invention, the particle size of the bioactive inorganic material is measured according to methods well known to those skilled in the art, and preferably by Particle Size Analyser or Scanning Electron Microscopy.

The powdery solid composition can comprise one or more bioactive inorganic materials having the above-mentioned features.

Step i) is carried out with a compaction pressure of at least about 2 MPa. During this step i), the powder of biocompatible resorbable organic polymer is deformed so as to optimize the contact surface between said biocompatible resorbable organic polymer and the bioactive inorganic material.

The compaction pressure is also called "mechanical stress" or "oedometric compression".

The compaction pressure depends in particular on the temperature implemented during step i). For example, a pressure of at least 2 MPa may be appropriate when step i) is carried out at a temperature higher than room temperature and around Tg.

Preferably, step i) is carried out with a compaction pressure of at least about 20 MPa, more preferably with a compaction pressure of at least about 40 MPa, and even more preferably with a compaction pressure of at least about 60 MPa. These minimum compaction pressures may be appropriate when step i) is carried out at room temperature.

Step i) can be performed with a compaction pressure of at most about 2000 MPa. This maximum pressure can avoid leaks.

In one preferred embodiment, step i) is carried out under vacuum. Vacuum enables removal of residues, water and/or air, avoids air porosities, and/or limits degradation.

Step i) is for example carried out below atmospheric pressure such as under medium or primary vacuum, and preferably at a pressure in the range of 0.01-20 mbar.

Step i) can be carried out from about 1 min to about 30 min, and preferably from about 1 min to about 5 min.

Step i) is generally performed at room temperature (i.e. 18-25°C). However, heating can also be implemented to manage the compaction pressure. In that case, the temperature may range from about 25°C to about 100°C. A temperature greater than 25°C can help to plastically deform polymer grains.

In one particularly preferred embodiment, step i) is carried out at a temperature of less than Tg. This embodiment can avoid the trapping of defects (air bubbles) that can adversely affect mechanical properties of the final product.

The powdery solid composition implemented in the compacting step i) comprises at least one biocompatible resorbable organic polymer and at least one bioactive inorganic material.

The bioactive inorganic material can represent from about 1% to about 70% by mass, preferably from about 10% to about 50% by mass, and more preferably from about 15% to about 40% by mass, with respect to the total mass of the powdery solid composition.

The biocompatible resorbable organic polymer can represent from about 30% to about 99% by mass, preferably from about 55% to about 90% by mass, and more preferably from about 60% to about 85% by mass, with respect to the total mass of the powdery solid composition.

The powdery solid composition can further comprise additives such as antioxidant additives, crosslinking additives, and/or biological additives.

Additives may represent from 0.001 to 20% by mass, with respect to the total mass of the powdery solid composition.

Step i) can be performed using Compressing Machine or Tensile-compression Machine.

Step i) of compacting is a solid step i). In other terms, it implements solid raw materials as the biocompatible resorbable organic polymer and the bioactive inorganic material and it leads to a solid material.

### Step i0)

The process can further comprise before step i), a step i0) of preparing said powdery solid composition.

Step i0) can be carried out by dry powder mixing process, compounding-cryogenic micronization process, or solvent-route mixing-solidifying-grinding process.

Dry powder mixing process can include mixing a powder of said at least one biocompatible resorbable organic polymer with a powder of said at least one bioactive inorganic material. This process of preparation is simple and leads to an homogeneous powdery solid composition which can then be directly used in step i) after drying.

In one particular embodiment, dry powder mixing process is carried out with a multidirectional mixer.

Dry powder mixing process can be carried out at room temperature or at a temperature ranging from about -60 to about 40°C.

Compounding-cryogenic micronization process can include at least the following sub-steps:
- extruding a mixture of a powder of said at least one biocompatible resorbable organic polymer and a powder of said at least one bioactive inorganic material said , so as to form an homogeneous solid extruded material, and
- cryomilling said extruded material.

Extrusion can be performed with an extrusion molding machine.

Extrusion is preferably performed at a temperature of at most about 150°C, and even more preferably of at most about 140°C.

Extrusion can lead to a composite filament which can then be cut into pellets.

Extrusion is preferably performed during at most 10 min, and more preferably during at most 5 min. Thus, hydrolytic degradation is prevented or at least reduced.

Cryomilling can be performed by means of a vibrational mill.

Cryomilling is preferably performed in liquid nitrogen.

Solvent-route mixing-solidifying-grinding process can include at least the following sub-steps:
- preparing a liquid composition comprising said at least one bioactive inorganic material, said at least one biocompatible resorbable organic polymer, and a solvent,
- solidifying said liquid composition, so as to obtain a solid material, and
- grinding said solid material to obtain powder.

The solvent to prepare the liquid composition can be selected from acetone, chloroform, tetrahydrofurane (THF), and mixtures thereof.

Solidification can be carried out by precipitation in a non-solvent organic polymer.

Preparation of the liquid composition and/or solidification of the liquid composition can be carried out at room temperature.

Grinding can be carried out by cryogenic grinding.

Compounding-cryogenic micronization process and solvent-route mixing-solidifying-grinding process are preferred. Indeed, they can lead to better dispersion of the bioactive inorganic material within the composite biomaterial, and better homogenization, and thus improved mechanical properties.

### Step ii)

The sintering step ii) is performed at a temperature Ts of at most Tg + 70°C. Said temperature Ts can ensure a welding of the biocompatible resorbable organic polymer powder.

A sintering step is well-known to be a step carried out in a solid state. In particular, the sintering step ii) is performed at a temperature Ts of at most the melting temperature of the biocompatible resorbable organic polymer (if a melting temperature of the biocompatible resorbable organic polymer exists). In other terms, the value of (Tg + 70°C) is in particular less than the value of the melting temperature of the biocompatible resorbable organic polymer (if a melting temperature of the biocompatible resorbable organic polymer exists).

Step ii) is advantageously carried out under oedometric conditions, preferably with no shearing.

This step ii) surprisingly leads to a composite biomaterial having improved mechanical properties, although it is carried out a low temperatures.

Besides, thanks to the use of such low temperatures, the degradation during processing of the composite biomaterial is avoided or at least very limited and no residual stress in the composite biomaterial is present.

Step ii) avoids or substantially reduces the hydrolytic, thermal, and thermo-oxidative degradation of the composite biomaterial during the process compared to processes of the prior art. Indeed, in classical processing such as injection molding, extrusion, or recently-described 3D-printing, elevated temperatures such as around 150-190°C are required and induce a lot of shear. Such elevated temperatures however immediately lead to unstability (degradation) of the biocompatible resorbable organic polymer and/or porosity issues (presence of defects).

The sintering temperature Ts of at most Tg + 70°C enables shaping the biocompatible resorbable organic polymer without residual stress or internal stress, and forming the sintered solid in the polymer rubbery state of said biocompatible resorbable organic polymer. At this sintering temperature Ts, the biocompatible resorbable organic polymer is able to be shaped without flowing.

In one preferred embodiment, step ii) is performed at a temperature Ts of at most about 130°C, more preferably of at most about 110°C, and even more preferably of at most about 80°C.

In one particularly preferred embodiment, the sintering step ii) is performed at a temperature Ts of at least Tg. Indeed, the sintering step at a temperature Ts of at least Tg enables polymer chains to diffuse at the interface between neighbouring grains.

Step ii) is generally performed at a temperature Ts of at least Tg + 10°C.

In one preferred embodiment, step ii) is performed at a temperature Ts of at least about Tg + 30°C.

Step ii) is carried out at a holding pressure greater than atmospheric pressure. The holding pressure ensures the contact surface between the powder grains and allow chains interdiffusion. In other terms, it keeps the interfaces in contact while the interdiffusion of polymer chains occurs at the interfaces.

In one preferred embodiment, step ii) is carried out at a holding pressure of at least 1 MPa.

With these holding pressures, the thickness of the compacted solid composition is maintained constant during the whole step ii).

In one preferred embodiment, step ii) can be carried out at a holding pressure of at most Pmax, where Pmax verifies the following relation: K * Pmax (in MPa) < Ts - Tg (in °C), where K ranges from 0.1 to 0.3 (in °C/MPa) (Ts and Tg being defined in the present invention).

Step ii) can be carried out at a holding pressure of at most 2000 MPa. This maximum pressure can avoid leaks.

The holding pressure is preferably less than the compaction pressure.

In one preferred embodiment, the sintering step ii) is carried out according to the following sub-steps:
- heating the compacted solid composition from room temperature to Ts with a heating rate ranging from about 1 to about 100°C/min, and preferably from about 5 to about 20°C/min, and
- maintaining Ts during at least about 1 min, and preferably at least 5 min.

Said embodiment enables better homogeneity of the bioactive inorganic material within the composite biomaterial.

Step ii) is preferably carried out under vacuum. Vacuum can be total or partial. In case of partial vacumm, air or helium can be used.

Step ii) is for example performed below atmospheric pressure, and more preferably at a pressure in the range of 0.1-500 mbar. Vacuum allows avoiding air porosities, and thus avoiding potential defects in the final composite biomaterial.

Step ii) is preferably carried out in an oven, and more preferaby in a convective oven.

Step ii) can be carried out from about 1 min to about 2880 min, and preferably from about 1 min to about 10 min. Surprisingly, such times used for step ii) enable entanglements of the polymer chains even if the organic polymer comprises long chains.

### Step iii)

Step iii) is a cooling step of the sintered solid, so as to form said composite biomaterial.

Step iii) can be carried out at a pressure equal or greater than atmospheric pressure. In one preferred embodiment, step iii) is carried out at atmospheric pressure.

In one preferred embodiment, step iii) is performed at room temperature (i.e. 18-25°C). The biocomposite material has in particular to reach a temperature below Tg before demolding.

Steps i), ii), and iii) of the process of the present invention are preferably solvent-free steps.

The process can further comprise a step iv) of sterilizing the composite biomaterial obtained in step iii) with ethylene oxide or X-ray, and preferably ethylene oxide.

Indeed, the composite biomaterial obtained in step iii) is free of residual or internal stress, and therefore it can be sterilized with significantly less aggressive methods than gamma-irradiation, such as ethylene oxide or X-ray sterilization.

Thanks to the process of the present invention, a composite biomaterial is obtained.

The composite biomaterial obtained according to the process as defined in the first object of the present invention is preferably in the form of a solid (massive) material. Consequently, it is preferably different from a powdery material.

The composite biomaterial is preferably resorbable.

As described above, the term "resorbable" means that the composite biomaterial has ability to degrade and be absorbed by the human or animal body. In other words, the composite biomaterial can be broken down or digested by microorganisms (e.g. bacteria, fungi, algae), especially by the action of enzymes. The reactions involved during biodegradation in humans or animals are hydrolysis reactions, that is to say the breaking of covalent bonds by reaction with water (cf. current standard NF EN 13432).

More particularly, the biocompatible organic polymer of the composite biomaterial should preferably have a molar mass higher than 40 000 g/mol, after being immersed in phosphate-buffered saline solution (PBS solution) under stirring at 37°C up to 120 days.

A second object of the present invention is a composite biomaterial comprising at least one biocompatible resorbable organic polymer and at least one bioactive inorganic material, wherein said composite biomaterial is obtained according to a process as defined in the first object of the present invention.

The composite biomaterial of the present invention is different from the materials of the prior art in that it displays a molar mass Mn (number-average molar mass) or Mw (weight-average molar mass) of the organic polymer well higher (e.g. weak hydrolytic and thermal degradation of the organic polymer thanks to the process as defined in the first object of the present invention), and consequently significantly improved mechanical properties after processing and over time of resorption.There is also no residual or internal stress in the composite biomaterial after processing.

More particularly, the composite biomaterial of the present invention comprises at least 30% by mass of said bioactive inorganic material with respect to the total mass of the composite biomaterial, and it displays a mechanical degradation marker MDM₁, such that MDM₁ = [UTS₁/UTS₀], in which UTS₁ represents the ultimate tensile strength in MPa of said composite biomaterial, and UTS₀ represents the ultimate tensile strength in MPa of a reference material obtained according to a same process as the one leading to said composite biomaterial but starting from a same biocompatible resorbable organic polymer which is neat, wherein MDM₁ ≥ 0.5.

In other words, said reference material is obtained when submitting a same biocompatible resorbable organic polymer without the presence of the bioactive inorganic material, to a same process of the invention (i.e. same conditions for steps i), ii), and iii)).

In the present invention, the ultimate tensile strength can be measured with a tensile test machine, preferably at room temperature and a loading step at 0,083 %/s (the % referring to the useful length of a test piece).

Preferably, MDM₁ ≥ 0.6, more preferably MDM₁ ≥ 0.7, and even more preferably MDM₁ ≥ 0.8.

In particular,
* the composite biomaterial comprises more than 5% by mass of said bioactive inorganic material with respect to the total mass of the composite biomaterial and said bioactive inorganic material has a particle size of less than or equal to 500 µm, or
* the composite biomaterial comprises more than 15% by mass of said bioactive inorganic material with respect to the total mass of the composite biomaterial,
and said composite biomaterial displays a polymer degradation marker PDM₁, such as DM₁ = [MW₁ / MW₀], in which MW₁ represents the weight-average molar mass in kg/mol of said biocompatible resorbable organic polymer extracted from said composite biomaterial, and MW₀ represents the weight-average molar mass in kg/mol extracted from a reference material obtained according to a same process as the one leading to said composite biomaterial but starting from a same biocompatible resorbable organic polymer which is neat, wherein DM₁ ≥ 0.8.

In other words, said reference material is obtained when submitting a same biocompatible resorbable organic polymer without the presence of the bioactive inorganic material, to a same process of the invention (i.e. same conditions for steps i), ii), and iii)).

Preferably, MDM₁ ≥ 0.85, and more preferably MDM₁ ≥ 0.9.

In one preferred embodiment, the composite biomaterial is devoid of residual or internal stress.

In the present invention, the presence or absence of residual or internal stress can be determined by WAXS (Wide angle X-ray Scattering) measurements, or by photoelastic measurements (e.g. photoelasticity measurements based on birefringence property).

Indeed, photoelasticity measurements and WAXS can be very useful to detect polymer chain alignment and the associated internal stress, and structural weakness in certain directions and dimensional instability. Interestingly, no residual stress was detected by photoelasticity measurements or WAXS in the composite biomaterial of the present invention.

Consequently, thanks to the process of the present invention, a composite biomaterial of the invention devoid of residual or internal stress is obtained, and can thus be sterilized with significantly less aggressive methods than gamma-irradiation, such as ethylene oxide or X-ray sterilization.

The bioactive inorganic material can represent from about 1% to about 70% by mass, preferably from about 10% to about 50% by mass, and more preferably from about 15% to about 40% by mass, with respect to the total mass of the composite biomaterial.

The biocompatible resorbable organic polymer can represent from about 30% to about 99% by mass, preferably from about 55% to about 90% by mass, and more preferably from about 60% to about 85% by mass, with respect to the total mass of the composite biomaterial.

The composite biomaterial as defined in the second object of the present invention or as produced according to the second object of the present invention has porosity of at most 2% by volume, and more preferably of at most 1% by volume. Consequently, it is not considered as a porous material. Then, porosity is created as soon as resorption occurs (i.e. during implantation).

A third object of the present invention is an implantable device wherein it comprises a composite biomaterial as defined in the second object of the present invention.

A fourth object of the present invention is a composite biomaterial as defined in the second object of the present invention, for its medical use.

A fifth object of the present invention is a composite biomaterial as defined in the second object of the present invention, for its use as a bone substitute or to support bone regeneration.

A sixth object of the present invention is a sterilization process, wherein said process comprises at least one step of sterilizing a composite biomaterial as defined in the second object of the present invention (or as obtained according the process as defined in the first object of the present invention) with ethylene oxide or X-ray, and preferably with ethylene oxide.

The present invention is illustrated in more detail in the examples below, but it is not limited to said examples.

### Examples

### Measurements

Scanning electron microscopy analysis (SEM) pictures were taken on uncoated samples with a Supra 55 microscope (Zeiss, Oberkochen, Germany). The samples were cryo-fractured in liquid nitrogen to observe the cross-section. Samples were observed at low acceleration voltage (1 kV).

X-ray tomography measurements were used to quantify the bioactive inorganic material particle dispersion into the biocompatible resorbable organic polymer matrix. They were conducted using a laboratory X-ray source on an EasyTom tomograph (RX Solutions, France) at high resolution, with a 1.5 µm voxel size, on small samples (~ 1.5×1×1mm). This tomograph was equipped with an LaB6 emission tip for the X-ray source, which ensured that the actual spot size was physically smaller than 1.5µm. This way, the resolution did not become modified by geometric blur. All the data were reconstructed by a filtered back-projection Feldkamp-algorithm.

The average molar masses, Mn (number-average molar mass) and Mw (weight-average molar mass) of neat PDLLA and the composite biomaterial after processing were determined by Size-exclusion chromatography (SEC) in Chloroform using a tetra detection system equipped with a Schimadzu SPD 20A (λ=280 nm), a Wyatt TREOS (3 angles), a ViscoStar II Wyatt and a Schimadzu RID 10A. The SEC system was equipped with three columns (PL gel 5µm Mixed C 300x7.5 mm, polystyrene/divinylbenzene). The running temperature was 30 °C. The dn/dc (which represents the change in the refractive index (dn) of a solution with respect to a change in its solute concentration (dc)) used for PDLLA in chloroform was 0.023 and the concentration of the samples was 3 mg/ml. Before the SEC analysis of composite biomaterials, the solutions were filtered with 0.45-µm filters to remove the bioactive inorganic material.

Tensile tests were performed with a tensile test machine at room temperature and loading step of 0.083%/s (tension mode), on dumbbell-shaped bars (with a gauge length l = 10 mm and width w = 4mm). They were punched out with a die from the disks.

The nature of crystalline phases formed at the surface of the samples after 2 weeks in phosphate saline buffer (PBS) was determined by X-ray diffraction (XRD). The measurements were performed on a Bruker Advance D8 diffractometer (Bruker AXS, USA), using CuKα radiation in a Θ-2Θ mode (starting and final 2Θ: 5 and 90°, step size: 0.039°, time per step: 7 s, operation parameters: 40 kV and 40 mA). The relative content of the crystalline phases and their crystal size was quantified by Rietveld analysis (Topas, Bruker, Madison, WI).

### Example 1: manufacturing of a composite biomaterial according to a process of the invention

An amorphous poly(D,L-lactide) hereafter referred to as PDLLA, with L-lactide content of around 82% by weight and D-lactide content of 18%, a density of 1.24 g.cm⁻³ and a glass transition temperature Tg of 56°C was supplied by Nature Works LLC (USA) (reference: PLA 4060D). PDLLA displays a weight-average molar mass Mw = 136 kg.mol⁻¹, a number-average molar mass Mn = 94 kg.mol⁻¹, and a polydispersity D_{M} = 1.4 obtained by SEC measurements in Chloroform. Before the process, PDLLA was micronized by Micronis (Colayrac-Saint-Cyrq, France) so as to have a particle size d₅₀ = 200 µm. PDLLA particles were then dried for 4 h at 45°C under vacuum.

Particles of 45S5 Bioglass^{®} from XL-Sci Tech, USA with a density of 2.64 g·cm⁻³ and an amorphous microstructure were used as the bioactive inorganic material. The bioglass has the following composition: SiO₂: 45% by mass; CaO: 24.5% by mass; P₂O₅: 6% by mass; and NaO₂: 24.5% by mass, with respect to the total mass of the bioglass. It has a particle size d₅₀ = 66 µm.

30 g of 45S5 Bioglass^{®} particles were mixed with 70 g PDLLA particles in a multidirectional mixer (Turbula type T2C, Basel, Switzerland) during 15 minutes so as to form a powdery solid composition comprising 30% by mass of bioglass with respect to the total mass of the powdery solid composition.

The powdery solid composition was then introduced into a sintering device installed on an Instron compressive testing machine (Instron, Norwood, MA, USA) equipped with a convection oven and maintained at a compaction pressure of 80MPa for 10 min, under vacuum (10 mbar) and at room temperature. The vacuum enables avoiding air porosities. During this step i), the powder of biocompatible resorbable organic polymer is plastic deformed so as to optimize the contact surface between said biocompatible resorbable organic polymer and the bioactive inorganic material.

Then, the resulting compacted solid composition is sintered in a convective oven with application of a holding pressure of 2MPa during the sintering step ii), so as to ensure the contact surface between the powder grains and allow chains interdiffusion.

The sintering step i) comprises heating the compacted solid composition from room temperature to 100°C with a heating rate of 5°C/min, and maintaining 100°C during 5 min.

Then, the sintered solid is cooled under a pressure of 2 MPa and at room temperature, until it reaches a temperature below Tg in order to prevent shrinkage cavities.

A sintered solid in the form of a disk with a diameter d = 25 mm, and a thickness h = 1 mm was obtained (composite biomaterial **CB1).**

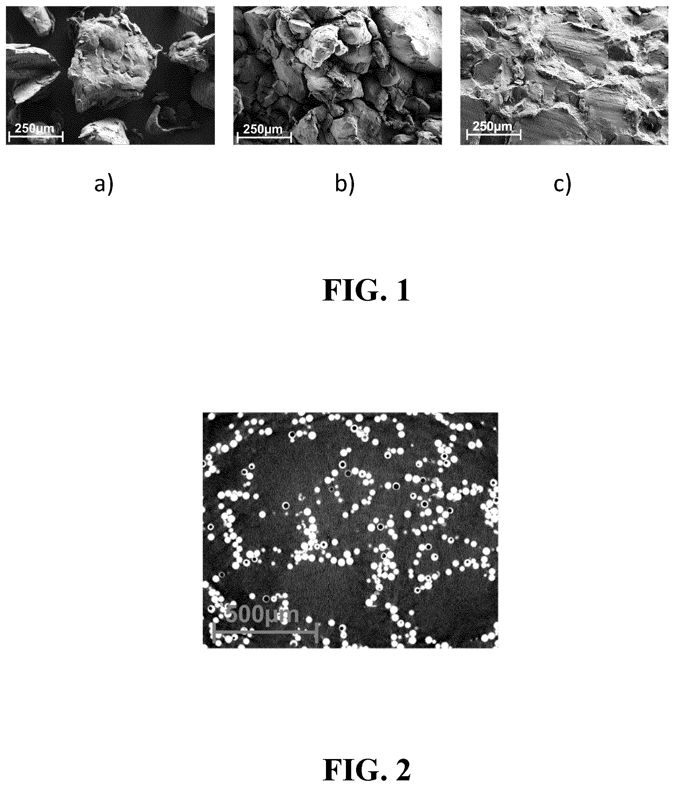

Figure 1 represents pictures obtained by scanning electron microscopy analysis (SEM) of the powdery solid composition (figure 1a), the compacted solid composition (figure 1b) and the sintered solid (figure 1c).

Since the powders are sintered under oedometric conditions with no shearing, the level of particle dispersion into the composite biomaterial after processing depends only on the level of powder mixing before sintering. As a result, thanks to the preparation of an homogeneous powdery solid composition, a composite biomaterial displaying an homogeneous inorganic particle distribution into the polymer matrix is obtained, while limiting particle agglomeration.

Figure 2 shows micro computed tomography (µCT) image of the obtained composite biomaterial.

### Example 2: manufacturing of comparative materials

The powdery solid composition as prepared in example 1 was submitted to injection molding (process not part of the invention) instead of sintering.

The injection molding was performed with a HAAKE Minijet injection molding machine (Thermo Fisher Scientific, Cleveland, OH, USA), using a cylinder temperature of 180°C and a mold temperature of 55°C under a pressure of 800 bar for 20 s. A injection molded composite biomaterial was obtained **(CB0).**

The sintered process as defined in example 1 was carried out with a same process as the one leading to **CB1** but starting from a same PDLLA which is neat (i.e. without 45S5 Bioglass^{®}), so as to provide a reference material **RM1.**

The injection molding process as defined in example 2 was carried out with a same process as the one leading to **CB0** but starting from a same PDLLA which is neat (i.e. without 45S5 Bioglass^{®}), so as to provide a reference material **RM0.**

The injection molded materials **(CB0, RM0)** and the sintered material **(RM1)** (materials not part of the invention) were compared to the sintered composite biomaterial **CB1** as obtained in example 1 (part of the invention) in terms of ultimate tensile strength, Young Modulus, weight-average molar mass, and number-average molar mass.

Table 1 below reports the values obtained.

**TABLE 1**

| **Material** | **UTS (MPa)** | **Young Modulus (MPa)** | **Mn (kg/mol)** | **Mw (kg/mol)** |
|---|---|---|---|---|
| **RM0** | 59.8 | 1460 | 83 | 118 |
| **CB0** | 29 | 1540 | 52 | 78 |
| **RM1** | 61.2 | 1460 | 91 | 127 |
| **CB1** | 37 | 1400 | 91 | 120 |

Remarkably, the UTS of **CB1** fabricated by sintering is much higher than that of **CB0** fabricated by injection molding.

Additionally, **CB0** fabricated by injection molding presented a clear PDLLA degradation with a loss of 35% of molecular weight. Interestingly, sintered **CB1** did not show a significant decrease of molecular weight. These results confirm that a low temperature manufacturing process ( sintering) leads to final parts with no sign of organic polymer matrix degradation.

Indeed, the reduction of organic polymer degradation was directly related to an increase of tensile strength. The high values of molecular weight after processing gives a promising alternative to injection molding and solvent techniques to obtain composite biomaterials with high mechanical properties, a good reproducibility and long resorption time during implantation. Moreover, sintering process can reduce the cost of the orthopedic implants fabrication since it is a low energy consuming process and there is a no-waste of raw material.

### Example 3: manufacturing of a composite biomaterial according to a process of the invention

An amorphous poly(L-lactide-co-D,L-lactide) medical grade acid terminaed with a 70:30 molar ratio of L-Lactide: D,L-Lactide hereafter referred to as PDLLA with a weight-average molar mass Mw of 800 000 g/mol, a number-average molar mass Mn of 580 000 g/mol and a polydispersity D_{M} = 1.4 was purchased to Evonik (Essen, Germany) (reference: RESOMER LR 708).

Before the process, PDLLA was micronized by Micronis (Colayrac-Saint-Cyrq, France) so as to have a particle size d₅₀ = 200 µm. PDLLA particles were then dried for 4 h at 45°C under vacuum.

Particles of 45S5 Bioglass^{®} from XL-Sci Tech, USA with a density of 2.64 g·cm⁻³ and an amorphous microstructure were used as the bioactive inorganic material. The bioglass has the following composition: SiO₂: 45% by mass; CaO: 24.5% by mass; P₂O₅: 6% by mass; and NaO₂: 24.5% by mass, with respect to the total mass of the bioglass. It has a particle size d₅₀ = 66 µm.

30 g of 45S5 Bioglass^{®} particles were blended with 70 g PDLLA particles in a Haake Minilab Rheomax CTW5 Mixing machine (Thermo Fisher Scientific, Cleveland, OH, USA) at low temperature (T = 140°C), a rotor speed of 40 rpm and with a short residence (~30 seconds), so as to form extrusion filament. Subsequently, the extrusion filament was cut into pellets using a mechanical granulation machine.

Then, the resulting pellets were micronizated with a vibrational mill Retsch MM400 (Retsch GmbH, Haan, Germany) which was equipped by two screw-type zirconium oxide jars, each one with a capacity of 50 ml. Prior each milling cycle, the jars were immersed in liquid nitrogen during 4 minutes. The grinding was carried out by vibrational shocks of one 12 mm diameter stainless ball in the jar during two times 3 minutes (2x3minutes) with a milling frequency of 30 Hz. A powdery solid composition comprising 30% by mass of bioglass with respect to the total mass of the powdery solid composition was obtained. The particle size obtained is d₅₀ = 280 µm.

The powdery solid composition was then introduced into a sintering device installed on an Instron compressive testing machine (Instron, Norwood, MA, USA) equipped with a convection oven and maintained at a compaction pressure of 80MPa for 10 min, under vacuum (10 mbar) and at room temperature. The vacuum enables avoiding air porosities. During this step i), the powder of biocompatible resorbable organic polymer is deformed so as to optimize the contact surface between said biocompatible resorbable organic polymer and the bioactive inorganic material.

Then, the resulting compacted solid composition is sintered in a convective oven with application of a holding pressure of 2MPa during the sintering step ii), so as to ensure the contact surface between the powder grains and allow chains interdiffusion.

The sintering step i) comprises heating the compacted solid composition from room temperature to 100°C with a heating rate of 5°C/min, and maintaining 100°C during 5 min.

Then, the sintered solid is cooled under a pressure of 2 MPa and at room temperature, until it reaches a temperature below Tg in order to prevent shrinkage cavities.

A sintered solid in the form of a disk with a diameter d = 25 mm, and a thickness h = 1 mm was obtained (composite biomaterial **CB2).**

In order to establish the bioactivity of the composite biomaterial of the invention, **CB2** was immersed in phosphate buffer for 2 weeks at 37°C under stirring, according to the same protocol as the one described by Kokubo et al. [Biomaterials, 2006, 27, 2907-2915], except that the simulated body fluid is replaced with phosphate saline buffer solution.

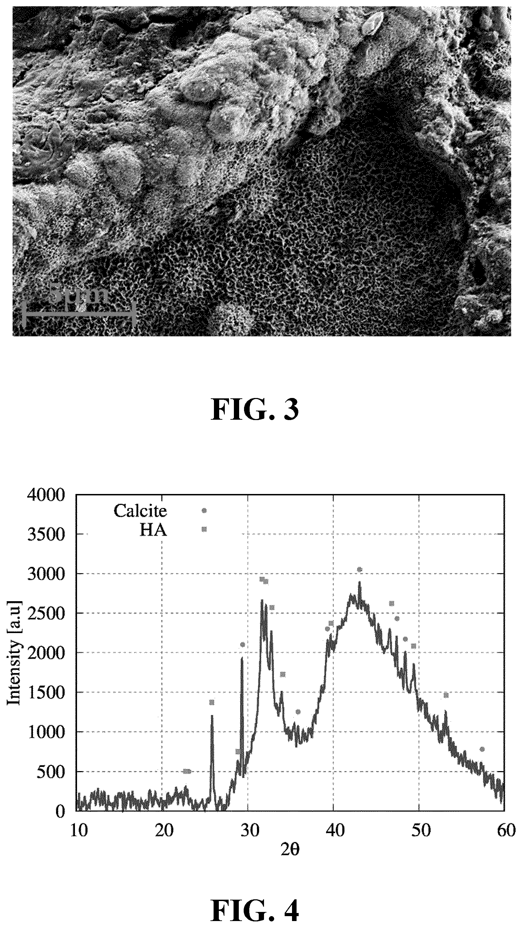

Figure 3 represents a picture of the composite biomaterial where a layer of carbonated apatite on the surface of said composite biomaterial was formed.

Figure 4 presents XRD patterns obtained on the composite biomaterial **CB2** surface after 2 weeks of immersion in PBS at 37°C. The diffraction peaks in the XRD pattern corresponded to the characteristic peaks of crystals of calcite (solid circles) and carbonated apatite (cHA) (solid squares). The carbonated hydroxyapatite (cHA) formation on the surface during the immersion study confirms the bioactive property of the composite biomaterial **CB2.**

### Example 4: photoelasticity measurement based on birefringence property

Gamma irradiation is the most widely used technique for orthopedic implants sterilization since it eliminates microorganisms, ensures good penetration and allows already-packaged products to be sterilized. However, in the case of PDLLA composites, this technique can significantly degrade polymer matrix and consequently decreases the mechanical properties and accelerates the biodegradation rate.

On the other hand, resorbable polyesters can be sterilized with ethylene oxide without affecting the molecular weight and mechanical properties. ethylene oxide sterilization is performed at 45-60°C with 65% of relative humidity and between 12 to 72 hours. With this sterilization conditions, ethylene oxide can act as a plasticizer of PDLLA, modify its structure and induce relaxation of residual stress.

Photoelastic measurement was used to evaluate residual stresses in the composite biomaterial **CB1** of the invention. This technique (as well as WAXS also called Wide angle X ray Scattering) can be very useful to detect polymer chain alignment and the associated internal stress. Moreover, during the implantation or in-vitro study, the plasticizer effect of water molecules can induce a stress relaxation of polymer chains and change the initial dimensions. Hence, photoelastic measurement (as well as WAXS study) may be used to identify structural weakness in certain directions and dimensional instability.

Since the photoelasticity study requires transparent materials, said study was made on the materials not containing the bioactive inorganic material **RM0** (injection molded material) and **RM1** (sintered material).

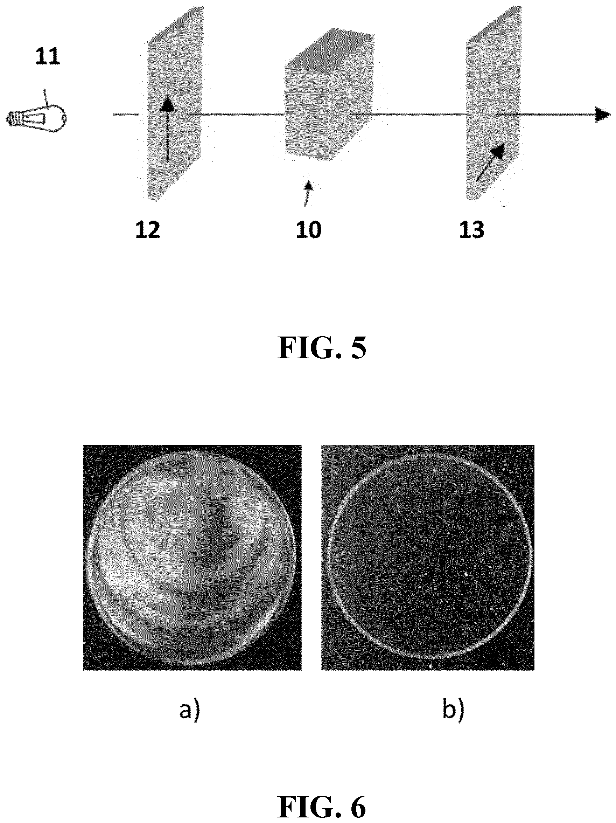

A figure 5 represents the device used to performed photoelasticity measurements based on birefringence property. In particular, photoelasticity makes it possible to detect possible residual stresses and/or anisotropies that could be found in parts after processing samples. When we observe a sample **10** with a light source **11** under crossed polarizers **12, 13,** no transmitted light is detected through the analyzer **13** if the sample is isotropic whereas a transmitted light is detected through the analyzer **13** if the sample is anisotropic.

**RM0** fabricated by injection molding (example 2) and **RM1** fabricated by sintering (example 1) were respectively placed between two crossed polarizers **12, 13** and the stress pattern was detected by a visible change in color and intensity in the sample.

Figure 6 shows the difference between the anisotropy of disks manufactured by injection molding (figure 6a) and thermal sintering (figure 6b). **RM0** (figure 6a) presented a residual stress (characterized by the isochromatic fringe pattern) due to the high shear rates and thermal gradient during the process. Interestingly, no stress was detected by the photoelastic study in **RM1** (figure 6b). Therefore, the biomaterial **RM1** may conserve the initial dimensions whereas the comparative biomaterial **RM0** may exhibit a contraction after the ethylene oxide sterilization that can be attributed to the polymer chains relaxation.

The same behaviour in terms of residual stress can be found if the study is carried out by WAXS on the materials containing the bioactive inorganic material **CB0** (injection molded material) and **CB1** (sintered material).

## Claims

1. A process for manufacturing a composite biomaterial comprising at least one biocompatible resorbable organic polymer and at least one bioactive inorganic material,
wherein said process comprises at least the following steps:
i) compacting a powdery solid composition comprising at least one biocompatible resorbable organic polymer and at least one bioactive inorganic material with a compaction pressure of at least 2 MPa, so as to form a compacted solid composition, said biocompatible resorbable organic polymer being defined by a glass transition temperature Tg, and having a cristallinity degree of at most 30%,
ii) sintering the compacted solid composition of step i) at a temperature Ts of at most Tg + 70°C and at a holding pressure greater than atmospheric pressure, so as to form a sintered solid, and
iii) cooling the sintered solid.

2. The process according to claim 1, wherein the bioactive inorganic material is selected from a bioactive glass, amorphous calcium phosphate, hydroxyapatite, tri-calcium phosphate, tetra-calcium phosphate, monocalcium phosphate, dicalcium phosphate, calcium silicate, and Mg alloy.

3. The process according to claim 1 or claim 2, wherein the biocompatible resorbable organic polymer is selected from aliphatic polyesters, aliphatic polycarbonates, gelatins, and collagens.

4. The process according to any one of the preceding claims, wherein the bioactive inorganic material represents from 1% to 70% by mass, with respect to the total mass of the powdery solid composition.

5. The process according to any one of the preceding claims, wherein step i) and/or step ii) are performed under vacuum.

6. The process according to any one of the preceding claims, wherein step ii) is performed at a temperature Ts of at most 130°C.

7. The process according to any one of the preceding claims, wherein step iii) is performed at room temperature.

8. A composite biomaterial comprising at least one biocompatible resorbable organic polymer and at least one bioactive inorganic material, wherein said composite biomaterial is obtained according to a process as defined in any one of the preceding claims.

9. The composite biomaterial according to claim 8, wherein said composite biomaterial comprises at least 30% by mass of said bioactive inorganic material with respect to the total mass of the composite biomaterial, and it displays a mechanical degradation marker MDM₁, such that MDM₁ = [UTS₁/UTS₀], in which UTS₁ represents the ultimate tensile strength in MPa of said composite biomaterial, and UTS₀ represents the ultimate tensile strength in MPa of a reference material obtained according to a same process as the one leading to said composite biomaterial but starting from a same biocompatible resorbable organic polymer which is neat, wherein MDM₁ ≥ 0.5.

10. The composite biomaterial according to claim 8 or 9, wherein said composite biomaterial comprises more than 5% by mass of said bioactive inorganic material with respect to the total mass of the composite biomaterial and said bioactive inorganic material has a particle size of less than or equal to 500 µm, or the composite biomaterial comprises more than 15% by mass of said bioactive inorganic material with respect to the total mass of the composite biomaterial, and
said composite biomaterial displays a polymer degradation marker PDM₁, such as DM₁ = [MW₁ / MW₀], in which MW₁ represents the weight-average molar mass in kg/mol of said biocompatible resorbable organic polymer extracted from said composite biomaterial, and MW₀ represents the weight-average molar mass in kg/mol extracted from a reference biocompatible resorbable organic polymer obtained according to a same process as the one leading to said composite biomaterial but starting from a same biocompatible resorbable organic polymer which is neat, wherein DM₁ ≥ 0.8.

11. The composite biomaterial according to any one of claims 8 to 10, wherein said composite biomaterial is devoid of residual or internal stress.

12. An implantable device wherein it comprises a composite biomaterial as defined in any one of claims 9 to 11.

13. A composite biomaterial as defined in any one of claims 9 to 11, for its medical use.

14. A composite biomaterial as defined in any one of claims 9 to 11, for its use as a bone substitute or to support bone regeneration.

15. A sterilization process, wherein said process comprises at least one step of sterilizing a composite biomaterial as defined in any one of claims 9 to 11 with ethylene oxide or X-ray.

## Patentansprüche

1. Verfahren zur Herstellung eines Verbund-Biomaterials, das wenigstens ein biokompatibles resorbierbares organisches Polymer und wenigstens ein bioaktives anorganisches Material umfasst,
wobei das Verfahren wenigstens die folgenden Schritte umfasst:
i) Kompaktieren einer pulverigen festen Zusammensetzung, die wenigstens ein biokompatibles resorbierbares organisches Polymer und wenigstens ein bioaktives anorganisches Material umfasst, mit einem Kompaktierungsdruck von wenigstens 2 MPa, so dass eine kompaktierte feste Zusammensetzung entsteht, wobei das biokompatible resorbierbare organische Polymer durch eine Glasübergangstemperatur Tg definiert ist und einen Kristallinitätsgrad von höchstens 30% aufweist,
ii) Sintern der kompaktierten festen Zusammensetzung von Schritt i) bei einer Temperatur Ts von höchstens Tg + 70°C und bei einem Haltedruck von mehr als Atmosphärendruck, so dass ein gesinterter Feststoff entsteht, und
iii) Abkühlen des gesinterten Feststoffs.

2. Verfahren gemäß Anspruch 1, wobei das bioaktive anorganische Material aus einem bioaktiven Glas, amorphem Calciumphosphat, Hydroxyapatit, Tricalciumphosphat, Tetracalciumphosphat, Monocalciumphosphat, Dicalciumphosphat, Calciumsilicat und einer Mg-Legierung ausgewählt ist.

3. Verfahren gemäß Anspruch 1 oder 2, wobei das biokompatible resorbierbare organische Polymer aus aliphatischen Polyestern, aliphatischen Polycarbonaten, Gelatinen und Collagenen ausgewählt ist.

4. Verfahren gemäß einem der vorstehenden Ansprüche, wobei das bioaktive anorganische Material 1 bis 70 Massen-% ausmacht, bezogen auf die Gesamtmasse der pulverigen festen Zusammensetzung.

5. Verfahren gemäß einem der vorstehenden Ansprüche, wobei Schritt i) und/oder Schritt ii) im Vakuum durchgeführt werden.

6. Verfahren gemäß einem der vorstehenden Ansprüche, wobei Schritt ii) bei einer Temperatur Ts von höchstens 130°C durchgeführt wird.

7. Verfahren gemäß einem der vorstehenden Ansprüche, wobei Schritt iii) bei Raumtemperatur durchgeführt wird.

8. Verbund-Biomaterial, das wenigstens ein biokompatibles resorbierbares organisches Polymer und wenigstens ein bioaktives anorganisches Material umfasst, wobei das Verbund-Biomaterial nach einem Verfahren erhalten wird, wie es in einem der vorstehenden Ansprüche definiert ist.

9. Verbund-Biomaterial gemäß Anspruch 8, wobei das Verbund-Biomaterial wenigstens 30 Massen-% des bioaktiven anorganischen Materials umfasst, bezogen auf die Gesamtmasse des Verbund-Biomaterials, und es einen mechanischen Abbaumarker MDM₁ aufweist, so dass MDM₁ = [UTS₁/UTS₀], wobei UTS₁ für die Zugfestigkeit des Verbund-Biomaterials in MPa steht und UTS₀ für die Zugfestigkeit eines Referenzmaterials in MPa steht, welches nach demselben Verfahren erhalten wird, das zu dem Verbund-Biomaterial führt, aber ausgehend von demselben biokompatiblen resorbierbaren organischen Polymer, das unvermischt ist, wobei MDM₁ ≥ 0,5 ist.

10. Verbund-Biomaterial gemäß Anspruch 8 oder 9, wobei das Verbund-Biomaterial mehr als 5 Massen-% des bioaktiven anorganischen Materials umfasst, bezogen auf die Gesamtmasse des Verbund-Biomaterials, und das bioaktive anorganische Material eine Teilchengröße von kleiner oder gleich 500 µm aufweist oder das Verbund-Biomaterial mehr als 15 Massen-% des bioaktiven anorganischen Materials umfasst, bezogen auf die Gesamtmasse des Verbund-Biomaterials, und
das Verbund-Biomaterial einen Polymerabbaumarker PDM₁ aufweist, so dass DM₁ = [MW₁ / MW₀], wobei MW₁ für das Gewichtsmittel der molaren Masse des aus dem Verbund-Biomaterial extrahierten biokompatiblen resorbierbaren organischen Polymers in kg/mol steht und MW₀ für das Gewichtsmittel der molaren Masse eines aus einem biokompatiblen resorbierbaren organischen Referenzpolymer extrahierten in kg/mol steht, welches nach demselben Verfahren erhalten wird, das zu dem Verbund-Biomaterial führt, aber ausgehend von demselben biokompatiblen resorbierbaren organischen Polymer, das unvermischt ist, wobei DM₁ ≥ 0,8 ist.

11. Verbund-Biomaterial gemäß einem der Ansprüche 8 bis 10, wobei das Verbund-Biomaterial frei von Restspannung oder innerer Spannung ist.

12. Implantierbare Vorrichtung, die ein Verbund-Biomaterial umfasst, wie es in einem der Ansprüche 9 bis 11 definiert ist.

13. Verbund-Biomaterial, wie es in einem der Ansprüche 9 bis 11 definiert ist, zur medizinischen Verwendung.

14. Verbund-Biomaterial, wie es in einem der Ansprüche 9 bis 11 definiert ist, zur Verwendung als Knochenersatz oder zur Unterstützung der Knochenregeneration.

15. Sterilisationsverfahren, wobei das Verfahren wenigstens einen Schritt des Sterilisierens eines Verbund-Biomaterials, wie es in einem der Ansprüche 9 bis 11 definiert ist, mit Ethylenoxid oder Röntgenstrahlung umfasst.

## Revendications

1. Procédé de fabrication d'un biomatériau composite comprenant au moins un polymère organique résorbable biocompatible et au moins un matériau inorganique bioactif,
dans lequel ledit procédé comprend au moins les étapes suivantes :
i) compacter une composition solide poudreuse comprenant au moins un polymère organique résorbable biocompatible et au moins un matériau inorganique bioactif avec une pression de compactage d'au moins 2 MPa, afin de former une composition solide compactée, ledit polymère organique résorbable biocompatible étant défini par une température de transition vitreuse Tg, et ayant un degré de cristallinité d'au plus 30%,
ii) fritter la composition solide compactée de l'étape i) à une température Ts d'au plus Tg + 70°C et à une pression de maintien supérieure à la pression atmosphérique, afin de former un solide fritté, et
iii) refroidir le solide fritté.

2. Procédé selon la revendication 1, dans lequel le matériau bioactif inorganique est sélectionné parmi un verre bioactif, du phosphate de calcium amorphe, de l'hydroxyapatite, du phosphate tricalcique, du phosphate tétracalcique, du phosphate monocalcique, du phosphate dicalcique, du silicate de calcium et un alliage de Mg.

3. Procédé selon la revendication 1 ou la revendication 2, dans lequel le polymère organique résorbable biocompatible est sélectionné parmi les polyesters aliphatiques, polycarbonates aliphatiques, gélatines et collagènes.

4. Procédé selon l'une quelconque des revendications précédentes, dans lequel le matériau bioactif inorganique représente de 1% à 70% en masse, par rapport à la masse totale de la composition solide poudreuse.

5. Procédé selon l'une quelconque des revendications précédentes, dans lequel l'étape i) et/ou l'étape ii) sont réalisées sous vide.

6. Procédé selon l'une quelconque des revendications précédentes, dans lequel l'étape ii) est effectuée à une température Ts d'au plus 130°C.

7. Procédé selon l'une quelconque des revendications précédentes, dans lequel l'étape iii) est réalisée à température ambiante.

8. Biomatériau composite comprenant au moins un polymère organique résorbable biocompatible et au moins un matériau inorganique bioactif, dans lequel ledit biomatériau composite est obtenu selon un procédé tel que défini dans l'une des revendications précédentes.

9. Biomatériau composite selon la revendication 8, dans lequel ledit biomatériau composite comprend au moins 30% en masse dudit matériau bioactif inorganique par rapport à la masse totale du biomatériau composite, et il présente un marqueur de dégradation mécanique MDM₁, tel que MDM₁ = [UTS₁/UTS₀], dans lequel UTS₁ représente la résistance à la traction maximale en MPa de ce biomatériau composite, et UTS₀ représente la résistance à la traction maximale en MPa d'un matériau de référence obtenu selon un même procédé que celui menant audit biomatériau composite, mais à partir d'un même polymère organique résorbable biocompatible qui est pur, dans lequel MDM₁ ≥ 0,5.

10. Biomatériau composite selon la revendication 8 ou 9, dans lequel ledit biomatériau composite comprend plus de 5% en masse dudit matériau bioactif inorganique par rapport à la masse totale du biomatériau composite et ledit matériau bioactif inorganique a une taille de particule inférieure ou égale à 500 µm, ou ledit biomatériau composite comprend plus de 15% en masse dudit matériau bioactif inorganique par rapport à la masse totale du biomatériau composite, et
ledit biomatériau composite présente un marqueur de dégradation du polymère PDM₁, tel que DM₁ = [MW₁ / MW₀], dans lequel MW₁ représente la masse molaire moyenne en kg/mol dudit polymère organique résorbable biocompatible extraite dudit biomatériau composite, et MW₀ représente la masse molaire moyenne en kg/mol extraite d'un polymère organique résorbable biocompatible de référence obtenu selon un même procédé que celui menant audit biomatériau composite mais à partir d'un même polymère organique résorbable biocompatible qui est pur, où DM₁ ≥ 0,8.

11. Biomatériau composite selon l'une quelconque des revendications 8 à 10, dans lequel ledit biomatériau composite est dépourvu de stress résiduel ou interne.

12. Dispositif implantable dans lequel il comprend un biomatériau composite tel que défini dans l'une quelconque des revendications 9 à 11.

13. Biomatériau composite tel que défini dans l'une quelconque des revendications 9 à 11, pour son usage médical.

14. Biomatériau composite tel que défini dans l'une quelconque des revendications 9 à 11, pour son utilisation comme substitut osseux ou pour soutenir la régénération osseuse.

15. Procédé de stérilisation, dans lequel ledit procédé comprend au moins une étape consistant à stériliser un biomatériau composite tel que défini dans l'une quelconque des revendications 9 à 11 avec de l'oxyde d'éthylène ou des rayons X.
